# EUROPEAN PATENT SPECIFICATION

(11) **EP 2 807 487 B1**
(45) Date of publication and mention of the grant of the patent: **29.11.2017**
(21) Application number: 13740630.2
(22) Date of filing: 25.01.2013
(51) Int. Cl.: G01N 33/569, C12Q 1/04, C07K 16/12

(54) **METHOD OF DETECTING TUBERCULOSIS**
VERFAHREN ZUM NACHWEIS VON TUBERKULOSE
PROCÉDÉ DE DÉTECTION DE LA TUBERCULOSE

(30) Priority: 27.01.2012 SE 1250055; 05.10.2012 SE 1251134
(43) Date of publication of application: 03.12.2014
(73) Proprietor: PEAS Institut AB, 587 23 Linköping (SE)
(72) Inventor: NAYERI, Fariba, 587 58 Linköping (SE)
(74) Representative: Brann AB
(86) International application number: PCT/SE2013/050057
(87) International publication number: WO 2013/112103

(56) References cited:
- WO-A1-2007/140545
- WO-A1-2009/094212
- WO-A1-2009/143565
- WO-A2-02/054073
- WO-A2-2006/125973
- WO-A2-2007/005627
- WO-A2-2007/136781
- WO-A2-2009/158521
- KOZBOR D ET AL: "The production of monoclonal antibodies from human lymphocytes", IMMUNOLOGY TODAY, ELSEVIER PUBLICATIONS, CAMBRIDGE, GB, vol. 4, no. 3, 1 March 1983 (1983-03-01), pages 72-79, XP023942945, ISSN: 0167-5699, DOI: 10.1016/0167-5699(83)90123-8 [retrieved on 1983-03-01]
- STREITZ MATHIAS ET AL: "Loss of receptor on tuberculin-reactive T-cells marks active pulmonary tuberculosis", PLOS ONE, PUBLIC LIBRARY OF SCIENCE, US, vol. 2, no. 8, 1 January 2007 (2007-01-01) , pages e735-1, XP009139526, ISSN: 1932-6203
- BIRKENMEIER G ET AL.: 'Production of conformation- specific monoclonal antibodies against alpha 2 macroglobulin and their use for quantitation of total and transformed alpha 2 macroglobulin in human blood' J IMMUNOL METHODS. vol. 162, no. 1, 04 June 1993, pages 59 - 67, XP023974352
- MOHRI H ET AL.: 'Anti- fibrinogen antibody mediates fibrinogen binding to platelet membrane glycoprotein Ilb-Illa' BR J HAEMATOL. vol. 85, no. 2, October 1993, pages 341 - 347, XP055081311

## Description

### FIELD OF THE INVENTION

The present invention relates to methods of detecting tuberculosis infection in an individual and of detecting whether an individual has been vaccinated against tuberculosis.

### BACKGROUND OF THE INVENTION

Tuberculosis (TB) is an infectious disease caused by *Mycobacterium tuberculosis* (Mtb).^{1,2} Presently, it is one of the leading causes of death from infectious diseases worldwide and thus poses a major public health problem. Even though the number of TB cases has been on the decline since 2006, an estimated 3 million death cases are reported annually out of the 8 to 10 million infected individual.^{1,3} About 2 billion individuals are latently infected with Mtb, and according to the WHO, it causes 9.8 million new cases and around 1.8 million deaths each year worldwide.^{1,3} The problem has been compounded with the emergence of multidrug resistant (MDR) and extensively drug-resistant (XDR) TB strains.^{2,4} The commonest method for diagnosing TB worldwide is sputum smear microscopy⁵, but in some developed countries it may be diagnosed by culture methods or rapid molecular tests.⁶ Looking at the pace at which the global epidemic of TB is increasing and the limited protection offered by the current vaccine, *M*. *bovis* BCG vaccine, there is a high demand for vaccine candidates that could arrest this public health issue.^{7,8} Consequently, lots of researchers are making efforts to develop new improved TB vaccine against Mtb.^{4,7}

The bacterium *Mycobacterium tuberculosis* (Mtb) was identified by Robert Koch in 1881 by culturing crushed granulomas. Mtb is an acid-fast, gram positive and slow-growing bacillus that measures about 0.5µm and 1-4µm in diameter and length respectively, making it smaller than many bacteria.⁹ Its waxy cell wall is composed of long chain fatty acids most of which are mycolic acids and glycolipids such as lipoarabinomannan (LAM), phenolic and phosphoglycoloipids, and sulpholipids. Other cell wall components include peptidoglycans and arabinogalactans.¹⁰ The nature of the cell wall enhances mycobacterial replication and persistence inside the macrophage and also useful in pre-adaptation inside the phagosome.^{9,11} Ability of Mtb to modulate antigen presentation and bactericidal activities of macrophages makes it the most successful human pathogen.¹¹

Mtb is an aerobic pathogen that lives in an intracellular compartment within the host and thus thrives better in the lung tissues where oxygen supply is fully adequate.³ It mostly affects the lungs (pulmonary TB) but can sometimes affect other sites (extra-pulmonary TB). The disease exclusively spreads in the air when pulmonary TB patients expel the bacilli through sneezing, coughing or speaking.⁶ The infectious tubercle bacilli maneuver their way into the body tissue through the respiratory tract after inhaling them from the air. They then establish themselves in the lung apex and lymph nodes after being carried by blood or lymph to these sites.³ The nature and outcome of the TB infection is primarily predicted by the interaction between the host and the pathogen at the onset of inflection.⁷ Lymphocytes and macrophages are recruited to the sites of infection within 2 to 6 weeks of infection leading to the development of granuloma, the hallmark of TB infection. The bacilli reside in the granuloma where they could get re-activated later or be released into the airways when their numbers incline.³

### Diagnosis of Tuberculosis; available methods

### Symptomatic diagnosis

Increased death tolls and repeated outbreaks of TB lead to increased awareness among people. They sought a medical advice if they had persistant cough (more than 2 weeks), chills, night sweats, anorexia, weight loss, fatigue, chest pain, and breathlessness. Extrapulmanory TB could have the symptoms like back pain, convulsions, neck lumps, red eye, dysuria, swollen foot, dizziness, infertility, joint pain, dysphagia, altered bowel habit, and skin ulcer.

### Culture analysis

Traditional methods of culture analysis are employed to identify the pathogenic bacteria. Sputum, gastric aspirate, liquid from lungs is squirted out through bronchoscopy with lavage procedure, tissue from lining of lungs through pleural biopsy and pleural fluid are the most common specimens that are cultured for a common diagnosis of TB disease. Acid fast staining is performed to identify the smear positive strains. Clearly well distinguishable colonies of Mtb could be obtained by culturing the specimens from suspects. Culture tests are sensitive (80%) and 98% specific accounting 2% false interpretetions.

### Tuberculin test/PPD

Tuberculin or Mantoux test or Purified Protein Derivative (PPD) or Priquette test is a common diagnostic tool for TB.

In PPD test, protein derived from mycobacterium is sterilized and purified from the culture concentrates, is injected intradermally in the forearm. Previous occurrence of the bacteria will result in immune reaction which could be identified by the swelling at the site of injection within 48-72 hours after the injection of tuberculin (glycerol extract of the tubercle bacteria). Induration (mounted hard area) at the site of injection is measured (5mm, 10mm or 15mm) and positivity is indicated if there is immune reaction.

HIV positive persons, patients with organ transplantation, immunosuppression, persons who had previous contact with mycobacterium or previous incidence of TB and persons living in poor sanitary/unclean areas are more prone for positive PPD results.

False positives are common with the cases of BCG vaccinated persons, previous exposure to TB infection and persons with very weak immune system while false negatives are observed for low CD4 T cells or HIV patients as there are chances of weak immune response to these tests.

### QuantiFERON-TB (QFT) Test

QFT-Gold TB test is more specific (98-99%) and sensitive (85-91%) test used in recent clinical diagnosis of TB.

This test is based on host immune response of secreting IFN-γ (interferon gamma) to mycobacterial secretory peptides such as ESAT-6 and CFP-10. Blood from suspects is collected and mixed with secretory peptides extracted from Mtb. IFN-γ produced in response to detection of Mtb antigens by the host immune cells is measured (OD at 450nm) to diagnose the TB infection.

### Chest X-rays

X-ray tests are taken to diagnose the severity of TB infection. The tests seem to be normal during initial stages of onset of the disease. Sometimes X-ray results could be misinterpreted due to similar symptoms of many other lung complications such as pneumonia, lymphoma, Wegener's granulomatosis, sarcoidosis, mesothelioma, osteoarthritis, bronchial carcinoma, empyema and also right heart failure.

Interaction between Mtb and macrophages as well as recruitment of immune cells to the site of infection and antigen presentation to the T cells are some of the most important determining factors of the progress of the infection.¹² A relatively small portion of individuals infected with Mtb go on to develop TB disease which is mostly human immunodeficiency virus (HIV) infected people.⁶

BCG vaccine was produced in 1921 by Albert Calmette and Camille Guerin at the Institute Pasteur in Lille, France. It is made up of live-attenuated strains of *M. bovis.*^{13,14} It has been the sole TB vaccine since then and lots of controversies have arisen concerning its efficacy, safety and overall impact on TB.⁷ This has raised a global public health concern about the need to develop new TB vaccine with improved efficacy and safety¹⁴ that could provide reliability with respect to preventing TB infection and arresting progression of the disease.

The BCG vaccine has been experimentally proven to give only 50 percent protection of the risk of developing TB. In adults, it offers incomplete and varying protection depending on other infections and geographical location.^{4,14} However, it has been shown to offer protection to children below five years of age against extrapulmonary forms of TB.¹⁵ BCG has little to no efficacy in preventing pulmonary TB and has also been contraindicated for use in immunocompromised patients and newborns with HIV.^{5,16} Several researchers have evaluated the differences in biological properties of BCG vaccine and associated them in explaining the varied detected degrees of protection. Some of these variations are linked to individual's previous exposure to environmental mycobacteria and TB pathogenesis such as exogenous re-infection.¹⁴

As it has become clearly evident that the use of BCG vaccine in vast number of individuals in many areas on the globe has not had much positive impact on the epidemiology of TB, it is generally accepted that a new vaccine candidate should be produced to diminish the global burden of TB.⁵ TB is curable but the rate at which individuals with active form of the disease spread it makes it extremely difficult to eradicate. It is estimated that a single person with active TB could infect at least 3 healthy individuals before receiving diagnosis and treatment.¹⁵ It has been predicted that there will be 101.7 million new cases and 17.9 million TB-related deaths in South Asia alone from 2015 through 2050 if no new vaccine is produced. This same research proves that, mass vaccination could prevent 85.9 million (84%) of these cases and 14.5 million (81%) of these deaths.^{1,2,5,6,15}

The new TB vaccines are being designed with multifunctional approach; safe as well as effective and prevent all forms of TB, in all age groups and among people with HIV.² Thus, there are pre-exposure vaccines that will protect individuals without TB infection and post-exposure vaccines to terminate the disease in already infected individuals. Presently, the majority of the vaccine candidates that are in clinical trials belong to the former.⁵

The primary role of a vaccine is to elicit a protective humoral immune response against infectious pathogens.¹⁷ Vaccination therefore involves the stimulation of the immune system to produce antibodies whose levels and activities are determining factors in the eradication of infection. Vaccination against intracellular pathogens like Mtb requires the induction of a strong cellular immune response.^{17,18} Hence, B cell biology is very crucial in elucidating the immune mechanisms for protection and vaccination against Mtb.^{14,18} The function of B cells and humoral immune response in protection against Mtb is complicated because of its intracellular localization. However, they are known to actively interact with the cellular immune components and shape the host's defense against Mtb. Apart from their roles in antigen presentation and antibody production, B cells modulate the differentiation of T cell and the development of cell-mediated immunity.¹⁷ Activated B cells also secrete large quantities of cytokines, notably, IFNγ and many interleukins (ILs), that are crucial in T cell-mediated immunity.^{14,17,18}

Advances in immunology in recent times have revealed the significance of natural immunity in disease treatment. In order to achieve great advancement in vaccine development, the interaction between the acquired cellular immune response and the bacterium from the onset of infection to the disease progression must be well understood.¹⁹ Immunity protects against Mtb progression.²⁰ Mtb-infected dendritic cells (DCs) and macrophages present Mtb-antigens to T lymphocytes via major histocompatibility complex (MHC) class I and to CD8+ cytotoxic T cells and CD4+ T helper (Th) cells via MHC class II resulting in the activation and proliferation of lymphocytes.²¹ T cells elicit immune response after Mtb infection by producing cytokines that in turn activate antimicrobial functions of DCs and macrophages and also by expressing their cytotoxic activities.¹¹ With regard to the cytokine environment, CD4+ T cells can elicit Th1 and Th2 responses. Th1 cells enhance cell-mediated immunity through cytotoxic T cells and macrophages whereas Th2 cells promote antibody production by B cells.²² It has been detected recently that CD8 T cells contain granulysin that directly attacks mycobacterium. In vivo studies with mice have shown that CD4 or MHC class II molecule negative mice cannot control Mtb replication when they are infected. Also, in vivo monoclonal antibody depletion of CD4 expressing T cells before Mtb infection renders mice prone to infection.¹² All T cell subtypes are needed in order to mount effective antimicrobial protection since they possess varied biological functions and thus, crucial in immunity against Mtb.²⁰

The role of antibodies in protection against Mtb infection has long been disregarded due to the notion that they are less effective in dealing with intracellular pathogens.²³ However, it has recently been proven, experimentally, that antibodies offer a lot of protective roles in diverse ways against Mtb and various researchers have used mAbs to reveal that antibodies possess the ability to regulate various aspects of mycobacterial infection to the host's advantage.²³ De Valliere and Co-workers¹⁷ have revealed that the activities of neutrophils and macrophages are enhanced in the presence of specific antibodies. They further demonstrated that Mtb are effectively processed and presented by DCs to CD4+ and CD8+ T cells when they are coated with specific antibodies. The role of antibodies in activating T cell immunity against intracellular pathogens has also been elucidated with the use of genital Chlamydia infection in antibody-deficient mice.²¹ In summary, antibodies function by attaching to the bacterial cells and stimulating immune processes that prevent bacterial growth. They can also produce immunomodulating effect on cellular immunity via cytokine signaling and inhibition of bacterial replication, neutralization of toxins, activation of the complement system, and by promoting antibody-dependent cellular cytotoxicity.¹⁷⁻²³

Memory B cells respond to specific antigen in second infection by producing antigen-specific antibodies.²⁴ B cells activation and differentiation during their stimulation and subsequent antibody production are two major areas in immunological research.²⁵ Antigen-specific antibodies are produced by antibody forming cells (AFCs) which develop within the first week of immune activation. AFC secrete IgM during the primary response and IgG in subsequent responses. Antibodies produced by AFCs have low affinity compared to those produced by memory cells during second exposure of antigens. However, there exists affinity maturation in antibodies produced by the former. The production of high affinity memory B cell population usually occurs within the germinal centers (GCs). There exists substantial increase in the number of antigen-specific B cells in the GC within the first two weeks after immunization and start to decrease afterwards.²⁵⁻²⁷ In a study conducted by Oshiba *et al.* on the affinity maturation of serum Ig, there was an enormous increase in high affinity IgG1 within the 7^{th} and 14^{th} day of immunization and started to decline until day 21 after which no affinity was observed.²⁶

Biacore SPR has made it possible to measure the binding of antigen to immobilized antibody without using labels.^{28,29} It enables direct and rapid determination of association and dissociation rates of binding process.²⁹ It has been employed in the study of a wide array of interaction partner molecules such as receptors, enzymes, drugs, growth factors, antigens, antibodies and many more.²⁸ The Biacore system from GE Healthcare³⁰ uses the phenomenon of surface plasmon resonance (SPR) to quantify interaction between molecules in real time.³⁰⁻³³ One of the interacting molecules (antibody) is firmly held to the surface of a sensor chip, that is coated with a gold film adhered to a surface matrix of carboxymethyl dextran, over which the other interaction partner (antigen) is passed.³² When there is an interaction between antibody and its specific antigen, an optical signal is generated as a result of change in mass and thus there is a change in refractive index. The interaction could be measured in response units (RU). Biacore has been used in many studies for detecting pathogens. Some of the research includes detection of Salmonella bacteria using immobilized antibodies reacting with *Salmonella* group A, B, D and E,²⁸ detection of *S. enteritidis* and *E*. *coli,*³⁴ and detection of serum antibodies using *Salmonella.*³⁵ WO2007/140545 relates to antibodies against Mycobacterium tuberculosis Elongation Factor-Tu for diagnosis and differentiation of M. tuberculosis infection in humans.

### Mimicry of host peptides a survival mechanism

An important mechanism underlying the strategies used by microbial pathogens to manipulate cellular functions is that of functional mimicry of host activities. In some cases, mimicry is achieved through virulence factors that are direct homologues of host proteins. In others, convergent evolution has produced new effectors that, although having no obvious amino-acid sequence similarity to host factors, are revealed by structural studies to display mimicry at the molecular level (Stebbins & Galan, 2001, Nature)
The immune system efficiently discriminates between self and non-self, however the occurance of autoimmune diseases indicates that such discrimination may be imprecise. Immunologic insults like exposure to pathogenic bacteria, viruses, aberrant expression of self proteins and exposure to cryptic antigens might trigger the immune reactions leading to autoimmune diseases. Antigenic epitopes present in pathogeous microorganisms, which resemble the host proteins can activate the cells of immune system, resulting in autoimmunity termed as molecular mimicry (Chodisetti et al 2012, BMC Immunology). Molecular mimicry has been demonstrated in T cell specific autoimmune diseases such as multiple sclerosis, myocarditis and diabetes. Tb has been associated with many different autoimmune diseases like SLE, and reumatoid arthritis suggesting that T cells can potentially recognize self antigens. T cells that cross-react with mycobacterial and self antigens are activated leading to autoimmune responses.
Alfa-2 macroglobulins (A2M) are boroad-spectrum inhibitors that play essential roles in host cell protection from parasitic or bacterial attack. The rearrangement of A2M upon cleavage of the bait region traps the attacking protease in a cage-like structure, thus rendering proteases secreted by infecting microorganisms ineffective. A2Ms are thus essential elements of innate immune system (Neves et al 2011).A2Ms are members of the same protein superfamily as components of the complement system, such as factor C3. Molecules of A2M superfamily must undergo major conformational changes upon activation and these events are crucial for their biological activities. Sequence analyses of a number of bacterial genomes have identified A2M-like genes in several bacteria, most of which are pathogenic species and/or colonize higher eukaryocytes. This observation reinforced the suggestion that bacteria, much like their eukaryotic counterparts, could employ A2M-like molecules to inhibit target proteases, thus facilitating the infection and colonization processes.

### SUMMARY OF THE INVENTION

Existing methods of diagnosing tuberculosis are associated with many problems. For example, culturing of samples from patients takes 4-8 weeks, and do not yield growth of mycobacterium tuberculosis if not taken from the infected region. Other available methods for detection of tuberculosis are PCR-based, or focused on T-cell mediated reactions, such as PPD and Quantiferon, and cannot distinguish a tuberculosis infected patient in need of treatment from a vaccinated individual having a high sensitivity. There are to our knowledge no known methods for following up the efficiency of therapy of tuberculosis or for evaluating the potency of vaccinations performed.

The present disclosure describes a method to produce specific antibodies against various Mycobacterium species. The present invention relates to an in vitro method for differentiating individuals having active Mycobacterium tuberculosis (Mtb) infection, individuals having a latent Mtb infection, and individuals being healthy or vaccinated against tuberculosis infection, comprising the steps-contacting a serum or plasma sample from said individual with an antibody produced by contacting heat-killed Mycobacterium tuberculosis bacteria with lymphocytes isolated from a host previously exposed to Mycobacterium tuberculosis bacteria, wherein the antibody is an IgG antibody, has specificity for a Mtb protein and shows cross-reactivity with a protein selected from the proteins of the following list:A2MG_HUMAN, FIBA_HUMAN, AACT_HUMAN, C03_HUMAN, FIBB_HUMAN, FIBG_HUMAN, A1AT_HUMAN, and APOA1_HUMAN; and- studying the interaction between the proteins in the sample and the antibody in comparison to healthy controls, wherein high levels of the proteins interacting with the antibody are indicative of an active Mtb infection, low levels of the proteins interacting with the antibody are indicative of a latent Mtb infection, and no levels of the proteins interacting with the antibody are indicative of a vaccinated or healthy individual.

The present invention also relates to an in vitro method for detecting whether an individual has a tuberculosis infection, comprising:- contacting a serum or plasma sample from the individual with an antibody having a specificity for Mycobacterium tuberculosis (Mtb), which antibody has been produced by contacting heat-killed Mycobacterium tuberculosis bacteria with lymphocytes isolated from a host previously exposed to Mycobacterium tuberculosis bacteria, wherein the antibody is an IgG antibody, has specificity for a Mtb protein and shows cross-reactivity with a protein selected from the proteins of the following list:A2MG_HUMAN, FIBA_HUMAN, AACT_HUMAN, C03_HUMAN, FIBB_HUMAN, FIBG_HUMAN, A1AT_HUMAN, and APOA1_HUMAN; and- studying the interaction between the proteins in the sample and the Mtb antibody in comparison to healthy controls, wherein the presence of an interaction between the proteins in the sample and the Mtb antibody indicates that the individual has a tuberculosis infection. In an embodiment, said method further comprises detecting whether the individual has been vaccinated against tuberculosis infection as defined by the independent claims, by:
- contacting the sample from the individual with an antibody having a specificity for the Bacille-Calmette-Guerin (BCG) vaccine, which antibody has been produced by contacting a heat-killed BCG vaccine with lymphocytes isolated from a host previously vaccinated with a BCG vaccine, wherein the antibody is an IgG antibody and
- studying the interaction between the sample and the antibody having a specificity for the BCG vaccine (i.e. the BCG antibody), in comparison to healthy controls,
wherein the presence of an interaction between the sample and the BCG antibody indicates that the individual has been vaccinated against tuberculosis infection.
There is also presented herein a method for detecting the potency or efficacy of a vaccination against tuberculosis infection in an individual, comprising:
- obtaining a sample from the individual,
- contacting the sample with an antibody having a specificity for the Bacille-Calmette-Guerin (BCG) vaccine, and
- studying the interaction between the sample and the BCG antibody,
wherein the presence of an interaction between the sample and the BCG antibody indicates potency or efficacy of the vaccination in the individual.
Said method may further comprise:
- contacting the sample with an antibody having specificity for Mycobacterium tuberculosis (Mtb), and
- studying the interaction between the sample and the Mtb antibody,
wherein the absence of an interaction between the sample and the Mtb antibody indicates potency or efficacy of the vaccination in the individual.
In one embodiment of the present invention, the above-described methods relate to detecting a latent tuberculosis infection. The sample obtained from the individual is a serum or plasma sample.
Further aspects are mentioned in the dependent claims.

In one embodiment, the Mtb antibody and/or the BCG antibody is immobilised to a solid surface and the interaction between the sample and the antibody is studied by use of surface plasmon resonance (SPR). However, interactions between sample and antibodies can be studied by any applicable technology, such as ELISA, lateral flow technologies and the like. Our study reveals the presence of antigenic particles in the blood long after infection or vaccination, respectively, which particles bind specifically to the antibodies produced herein.

### SHORT DESCRIPTION OF THE DRAWINGS

**Fig. 1** **A**-**C:** Graphs showing the specificity of the antibodies produced against Mtb and BCG in binding to negative and positive controls.
**Fig. 2****:** Graph showing the responses of TB patients (n=5) to the various channels. P*=Patient. BCG1=BCG channels 1 (blue colour), BCG2=BCG channel 2 (red colour) and Mtb= Mtb channel (green colour).
**Fig. 3A****-D:** Graphs showing the number of positive and negative culture and biacore results.
**Fig. 4****:** Histogram showing the binding signal to flow cells immobilised by specific antibodies.

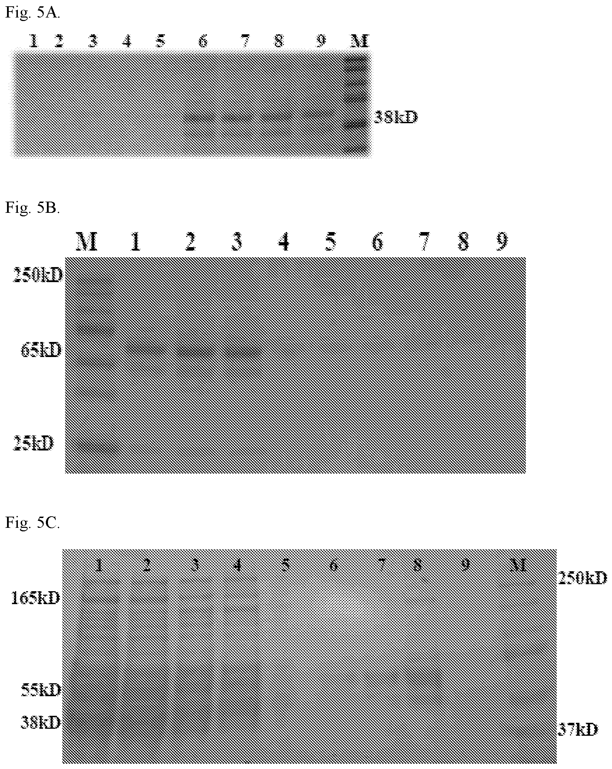

**Fig. 5****:** Identification of protein antigens in albumin/IgG depleted serum samples of TB patients and healthy individuals by SDS-PAGE. (a) lanes 1-5 are TB samples to which Mtb antibodies were added and incubated for 1 hour, lanes 6-9 are TB samples without antibodies and M represents molecular marker reference. (b) lanes 1-3 are TB samples without antibodies, lanes 4-9 are TB samples to which Mtb antibodies were added and incubated for 1 hour and M represents molecular marker reference. (c) lanes 1-4 are samples of two TB patients , 5-9 are samples of healthy individuals and M is a molecular marker reference. All samples were subjected to the well at 20µl per lane and gel was set to run at 200V for about 1-2 hour.

### DETAILED DESCRIPTION OF THE INVENTION

We have developed specific antibodies against Mycobacterium species (PPD, BCG and MTB) in vitro. The antibodies immobilized in a biosensor Surface Plasmon Resonance (SPR) system could recognize particles in serum/plasma of TB patients with high sensitivity and specificity.

This observation could be the basis for development of a diagnostic test to
- diagnose TB infection in patients
- Differentiate the various mycobacterial infections from each other (M. tuberculosis, M. Bovis, non tuberculosis mycobacterial infections., etc)
- Monitor the therapy in TB patients and function as a potency test for BCG vaccines.
- Identification of particles found in the blood in patients or vaccinated individuals might be used as a future candidate for a safe vaccine production against tuberculosis.

### Materials and methods

### Sample collection

A total of 36 individuals were used in this study; 23 males and 13 females between the ages of 18 and 85 years. Pneumonia samples (n=11) were taken from patients who were diagnosed of pneumonia and treated at the Department of Infectious Diseases, Linkoping University hospital, between 1997 and 1999. Healthy control samples (n=20) and TB samples (n=5) were also obtained from patients diagnosed of TB at the same hospital. Antibodies were produced from blood of a patient who had MDR TB in May 2011 and got treated at that time. His blood sample was taken for this study when he re-visited the hospital in September 2011. Ethical permission was provided by the local ethical committee at Linköping University Hospital at each instance.

### Production of antibodies against bacteria

Blood from TB patients was collected into EDTA tube using EDTA as anticoagulant. After collection, 5 ml of blood was diluted with equal volume of physiological (0.9%) NaCl in 15 ml centrifuge tube and carefully layered over 10 ml Lymphoprep solution (Fresenius Kabi Norge AS, Norway) in a 50 ml centrifuge tube (care was taken not to mix the layers). The resulting gradient was centrifuged at 800g for 20 minutes at 20°C. Three bands were obtained. The intervening band containing mononuclear lymphocytes was carefully drawn out with Pasteur pipette without mixing the layers. Lymphocytes were cultured in Leibovitz L-15 medium containing 10% FBS. After 24 hours of incubation (37 °C), 5µl of either heat-killed (kept in water bath at 80 °C for 20 minutes) BCG vaccine (Statens Serum Institute, Denmark) or Mtb was added to the medium. Light microscope was used to view morphological changes of lymphocytes in the medium, each day, throughout the period of incubation and pictures of cells were taken with 1.3M pixels digital camera (Microstone Infotech INC.) connected to the microscope and PC. Pictures were collected on PC with the help of Minisee software (ScopeTech, China). After 2 weeks of incubation, the culture medium was centrifuged at 400g for 10 minutes to settle down the cells and then was filtered using 0.45µm cellulose acetate sterile filters (Whatman, Schleicher & Schuell, Germany). The filtrate was centrifuged again at 400g for 70 minutes in 100kDa Amicon Ultra centrifugal filter devices. Antibodies, having large molecular weights, were left behind on the filter while other substances were drained as filtrate. Antibodies collected from the filter were preserved at -20°C by adding 5µl physiological (0.9%) NaCl solution.

### SDS-PAGE

SDS-PAGE was performed with MINI-PROTEAN precast gels (BIO-RAD, Sweden) to determine the molecular weight of the antibodies. Antibody samples were diluted with PBS (pH 7.4, Apoteket AB) and equal volume of loading dye was added. Samples were diluted in a ratio of 1:1 with laemmli sample buffer and heated in the water bath at 98 C for 5 minutes to denature proteins before loading into the wells. 200kDa Precision Plus Protein Standard Marker (BIO-RAD, Sweden) was used as reference. 20µl of samples and marker were loaded in each well and the gel was set to run at 200V for about an hour.

### Liquid chromatography-tandem mass spectrometry (LC-MS/MS)

### - In-gel digestion of proteins

Protein bands of interest were cut and placed into 1.5ml capped micro centrifuge tubes. 150µl of solvent A (25mM NH₄HCO₃ in water mixed with equal volumes of acetonitrile, pH 8.0) was added to each gel piece in vial and were vortexed occasionally for 10 min. The solution was discarded and the procedure was repeated 3 times until color of the gel disappeared. Gel pieces were dried in a vacuum centrifuge (Savant Instrument Inc. Speed Vac SC100) for 30 min. 60µl of 10mM DTT in 25mM NH₄HCO₃ was added to reduce the protein and then was incubated at 56°C for 1 hour. Gel pieces were cooled to RT and DTT solution was replaced with 70µl of 55mM iodoacetamide in 25mM NH₄HCO₃ and was incubated at RT for 45 min in the dark. Gel pieces were washed with 100µl of 25mM NH₄HCO₃ for 10 min, dehydrated twice with 100µl of solvent A and then dried completely in vacuum centrifuge. Gel particles were rehydrated with 60µl 0.005mg/ml trypsin (Sequence Grade Modified, Promega) solution in 25mM NH₄HCO₃ and incubated at 37°C for 24 hours. 50µl of water was added and the resulting solution (peptide extract) was transferred into a new 1.5ml microcentrifuge after 20 min. Two additional extractions of the gel pieces were done with 50µl of 5% TFA in 50/50 water/acetonitrile for 20 min each. Peptide extracts were dried completely in vacuum centrifuge and stored at -20°C until LC-MS/MS analysis.

### - LC-MS/MS analysis

Peptides were analyzed by LC-MS/MS. Separation was done using nano-flow HPLC system (EASY-nLC from Bruker Daltonics, Bremen, Germany) and data were retrieved using on-line electrospray ionization ion trap, HCT-ultra PTM Discovery System (Bruker Daltonics, Bremen, Germany). Peptides were separated by reverse phase chromatography on a 20 mm×100µm (particle size 5µm) C18 pre column followed by a 100 mm×75 µm C18 column (particle size 5µm, Nanoseparations, Netherlands) at a flow rate 300 nL/min.
Automated online tandem MS analyses were performed by using alternating collision induced dissociation (CID) of peptide ions. Peak lists were created from the raw data using Bruker Daltonics Data Analysis 3.4 (Bruker Daltonics, Bremen, Germany) and the Mascot Generic Format (MGF) files produced were used to search for proteins in Swiss-Prot (UniProtKB/Swiss-Prot Release 2011_03) on public Mascot server (www.matrixscience.com) (Table IX).
Search parameters allowed mass errors up to 0.6 Da for MS data, and up to 0.6 Da for MS/MS data in case of CID of peptide ions. Peptides charge states were varied; 3 missed cleavage sites were allowed. Cysteine carbamidomethylation was selected as a fixed modification. N-terminal protein acetylation and methionine oxidation were selected as variable modifications. Identification of peptides was considered reliable if the MS/MS spectra had Mascot scores above 25.

### Immobilization of antibodies

Biacore 1000 SPR biosensor instrument (GE Healthcare, Uppsala, Sweden) was used to detect antigen-antibody interaction. Antibodies prepared were immobilized onto the flow cells of CM5 sensor chip using amine coupling method.³⁶ The sensor surface of the chip coated with carboxymethylated dextran layer was activated using equal volumes of N-ethyl-N'-(dimethylaminopropyl) carbodiimide hydrochloride (EDC) and N-hydroxysuccinimide (NHS) mixture. NHS esters produced upon activation of chip react with the ligands that contain primary amino groups. The unreacted NHS esters were deactivated with Ethanolamine. Each channel of the chip was immobilized with antibodies (20µl) diluted with acetate pH 4.5.³⁴

**Table I: Dilution specifications for sample injections during immobilization of CM5 sensor chip.**

| S.no | Content | Dilutions | Injection volume (µl) | Injection time |
|---|---|---|---|---|
| 1. | NHS+EDC | 100 µl+100 µl | 35 µl | 7 minutes |
| 2. | Antibodies | 20µl in 80µl Acetate 4.5 | 35 µl | 7 minutes |
| 3. | Ethanolamine | 70 µl | 35 µl | 7 minutes |

### Testing specificity of antibodies

Each channel was tested for the specificity of antibodies immobilized on it before running serum and plasma samples. Antibodies produced *in vitro* against Mtb and BCG vaccine were immobilized individually to one channel at a time in Biacore 1000 instrument. Anti-human IgG and the bacteria (against which the antibody was immobilized on that particular channel) were used as positive controls. Negative controls were anti-guinea pig IgG and other bacteria.

### Running Samples and detection of bacteria

200 µl of each TB serum/plasma sample was centrifuged in 100kDa Amicon Ultra centrifugal filter at 400g for 10 minutes. The pellets were diluted in the ratio of 1:20 with PBS (pH 7.4, Apoteket AB) and injected at a flow rate of 5µl/min for 3 minutes. Surface of the sensor chip was washed with regeneration buffer (equal volumes of 1M sodium hydroxide and glycine (pH 2.0)) at a flow rate of 5µl/min for 1 minute. The unbound molecules were removed during the running process by using HEPES buffered Saline (HBS) buffer (10mM HEPES, pH 7.4. 150mM NaCl, 3.4mM EDTA, 0.005% Biacore surfactant P20). Angular deflection of the SPR signal is used for determining the interaction of bacteria (antigen) with the antibody coated on the channel and the response was measured in response units (RU).

**Table II: Specifications of dilutions during a single run in Biacore.**

| Content/ID | Dilutions | Injection volume (µl) |
|---|---|---|
| Antibodies | 20µl in 80µl PBS | 15µl |
| Regeneration buffer | 1:1 ratio of 1M NaCl and glycine 2.0 | 5µl |
| TB (plasma and serum) | 1µl in 20µl PBS | 15µl |
| Bacteria | 20µl in 80µl PBS | 15µl |
| Pneumonia (plasma and serum) | 1µl in 20µl PBS | 15µl |

### Results

### Culturing B cells

Different sizes of B cells were observed in the L-15/FBS medium before and after stimulation with heat killed bacteria. Cells were also seen to aggregate in the medium with bacteria while cells in the medium without bacteria were seen as individual cells with distinct nuclei (pictures not shown). These observations were true for all cultures.

### SDS PAGE

Molecular weight of antibodies produced was determined by running SDS-PAGE. Bands were obtained at 50kDa (heavy chain) and 25kDa (light chain) for various dilutions of antibodies (1:25, 1:50, 1:75, and 1:100) with PBS (pH 7.4) (data not shown).

### Immobilization of antibody on chip

The CM5 sensor chip was immobilized with antibodies produced from BCG vaccine (two channels) and Mtb (one channel) stimulated B-cells. (Tables III-V).

**Table III: Response obtained for Immobilization of BCG1 antibodies.**

| S. no | Content/ID | Dilutions | Injection volume (µl) | Response (RU) |
|---|---|---|---|---|
| 1. | NHS+EDC | 100 µl+100 µl | 35 µl | 226.1 |
| 2. | Antibodies | 20µl in 80µl Acetate 4.5 | 35 µl | 987.6 |
| 3. | Ethanolamine | 70 µl | 35 µl | 727.1 |

**Table IV: Response obtained for Immobilization of BCG2 antibodies.**

| S. no | Content | Dilutions | Injection volume (µl) | Response (RU) |
|---|---|---|---|---|
| 1. | NHS+EDC | 100 µl+100 µl | 35 µl | 89.4 |
| 2. | Antibodies | 20µl in 80µl Acetate 4.5 | 35 µl | 1056.2 |
| 3. | Ethanolamine | 70 µl | 35 µl | 741.7 |

**Table V: Response obtained for Immobilization ofMtb antibodies.**

| S. no | Content | Dilutions | Injection volume (µl) | Response (RU) |
|---|---|---|---|---|
| 1. | NHS+EDC | 100 µl+100 µl | 35 µl | 244.0 |
| 2. | Antibodies | 20µl in 80µl Acetate 4.5 | 35 µl | 4366.4 |
| 3. | Ethanolamine | 70 µl | 35 µl | 2402.5 |

### Specificity test of the chip

A specificity test was performed for each of the channels after the immobilization steps. Anti-guinea pig IgG and other bacteria were used as negative controls and anti-human IgG, Mtb and BCG vaccine were used as positive controls.

### Sample Run

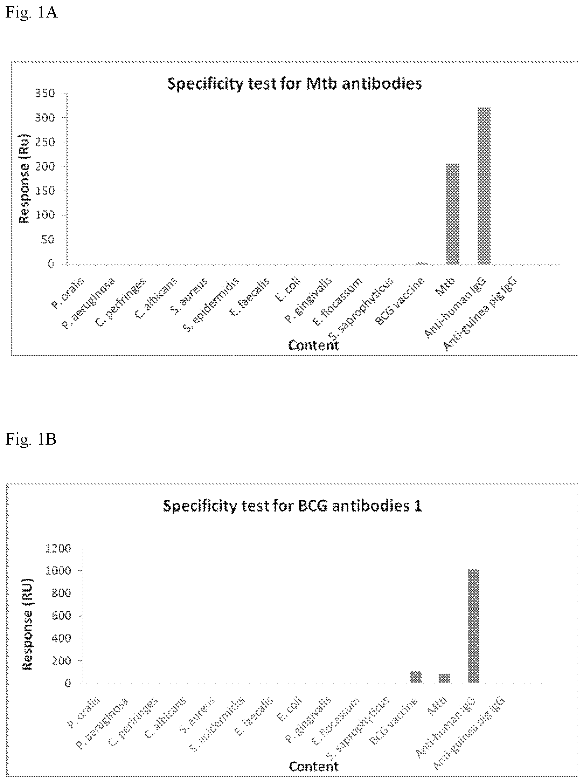

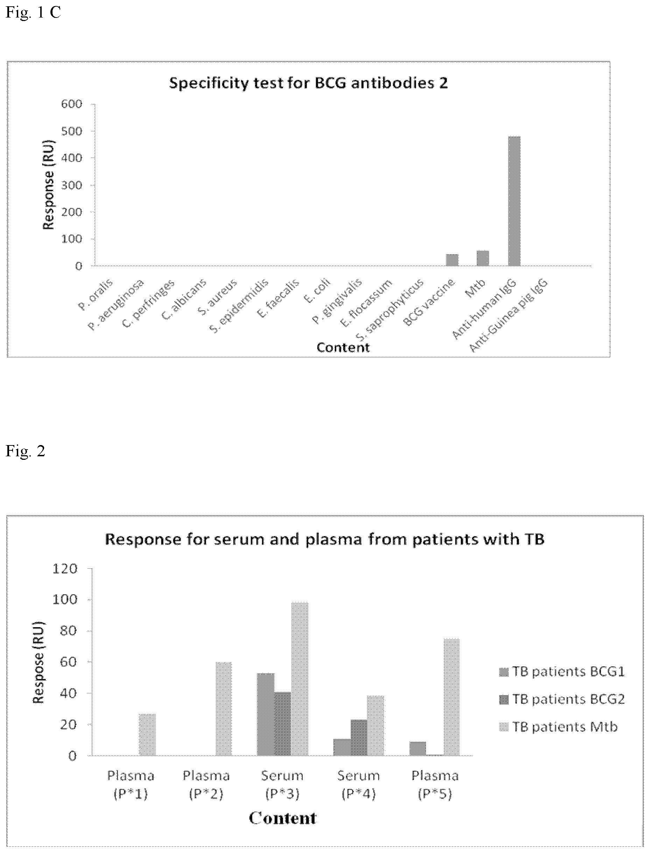

Plasma and serum from healthy subjects and TB patients were run in Biacore 1000 instrument and the results obtained were shown in Fig. 1A-C, illustrating the specificity of the antibodies produced against Mtb and BCG in binding to negative and positive controls. **Fig. 1A** shows the results of the specificity test for the Mtb antibodies: a very weak response to BCG and strong responses to Mtb and anti-human Ig; no response to the negative controls (i.e. other types of bacteria and anti-guinea pig IgG). **Fig. 1B** and **Fig. 1C** show the results of the specificity test for the BCG antibodies (two channels): weak responses to BCG and Mtb and a strong response to anti-human IgG; no response to the negative controls. All the healthy samples showed negative response.

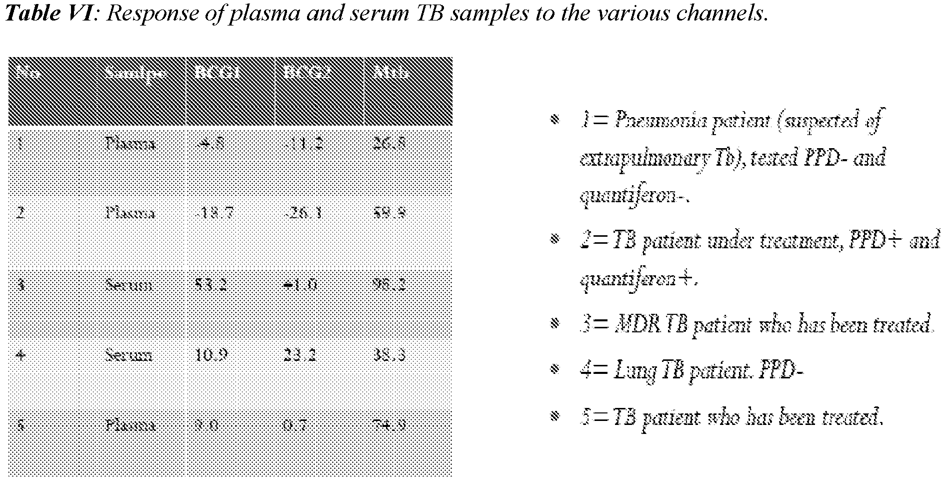

**Figure 2****:** Graph showing the responses of TB patients (n=5) to the various channels. P*=Patient. BCG1=BCG channels 1 (blue colour), BCG2=BCG channel 2 (red colour) and Mtb= Mtb channel (green colour).

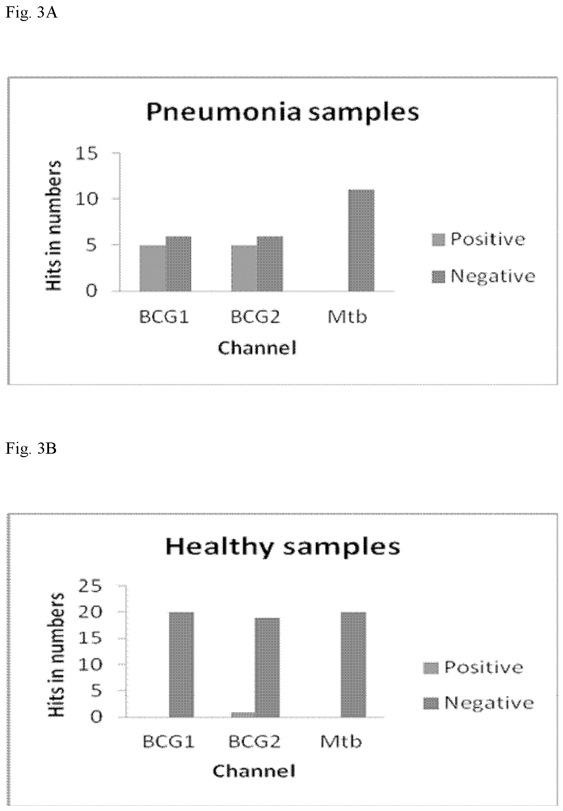

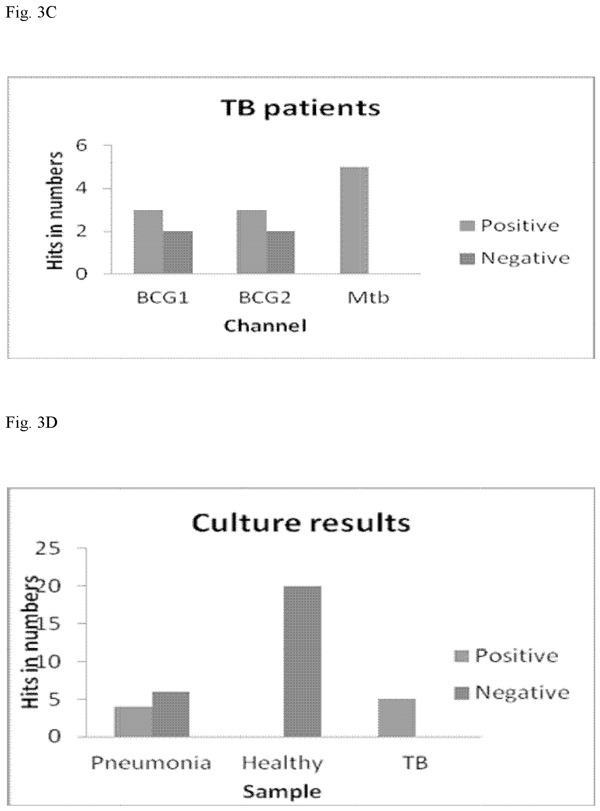

**Fig. 3** **A**-**D:** Graphs showing the number of positive and negative culture and biacore results. A) All eleven pneumonia samples responded negative on Mtb channel (red colour), five responded positive (blue) and six responded negative (red) on both BCG channels. B) All twenty healthy samples responded negative to Mtb channel and BCG channel 1(red), two sample responded positive (blue) and eighteen negatives (red) on BCG channel 2. C) All five TB samples responded positive to Mtb channel (blue) but three (blue) and two (red) showed positive and negative response on each BCG channel respectively. D) four out of eleven pneumonia patients were culture positive, all healthy samples were culture negative and all TB patients were culture positive.

**Table VII : Table showing clinical information, culture and Biacore results of pneumonia patients. B1=BCG channel 1, B2=BCG channel 2, Mtb=Mtb channel.**

| Clinical information and culture results | | | | | | | | | Biacore results | | |
|---|---|---|---|---|---|---|---|---|---|---|---|
| **No.** | **Sex** | **Age** | **Admitted** | **Diagnosed of** | **Chronic lung disease** | **Smoking** | **Culture** | **Dead** | **B1** | **B2** | **Mtb** |
| **1.** | **Male** | **71** | **97/10/13** | **Pneumonia** | **No** | **Yes** | **Negative** | **No** | **Neg** | **Neg** | **Neg** |
| **2.** | **Male** | **51** | **97/10/22** | **Pnc pneu** | **Yes** | **Yes** | **Pnc NPH** | **No** | **Pos** | **Neg** | **Neg** |
| **3.** | **Female** | **74** | **97/11/18** | **Pneumonia** | **No** | **Nil** | **Negative** | **No** | **Neg** | **Neg** | **Neg** |
| **4.** | **Male** | **47** | **97/10/24** | **Pneumonia** | **No** | **No** | **Negative** | **No** | **Pos** | **Pos** | **Neg** |
| **5.** | **Male** | **84** | **97/10/26** | **Pneumonia** | **No** | **Nil** | **Pnc NPH** | **No** | **Pos** | **Pos** | **Neg** |
| **6.** | **Male** | **80** | **98/04/08** | **Pneumonia** | **No** | **No** | **Negative** | **No** | **Neg** | **Pos** | **Neg** |
| **7.** | **Male** | **33** | **99/11/02** | **Mycoplasma** | **No** | **No** | **Nil** | **No** | **Neg** | **Neg** | **Neg** |
| **8.** | **Male** | **34** | **97/11/07** | **CMV pneu** | **No** | **Nil** | **Aspergillo** | **No** | **Pos** | **Pos** | **Neg** |
| **9.** | **Male** | **84** | **98/03/20** | **Pneumonia** | **No** | **No** | **Negative** | **No** | **Pos** | **Pos** | **Neg** |
| **10.** | **Female** | **30** | **98/04/24** | **Pneumonia** | **No** | **No** | **Pnc NPH** | **No** | **Neg** | **Neg** | **Neg** |
| **11.** | **Male** | **42** | **97/10/17** | **Pneumonia** | **No** | **No** | **Negative** | **No** | **Neg** | **Neg** | **Neg** |

**Table VIII: Biacore results for healthy controls.**

| Healthy Samples | Biacore results | | |
|---|---|---|---|
| | BCG channel 1 | BCG channel 2 | Mtb channel |
| 1 | Negative | Negative | Negative |
| 2 | Negative | Negative | Negative |
| 3 | Negative | Negative | Negative |
| 4 | Negative | Negative | Negative |
| 5 | Negative | Negative | Negative |
| 6 | Negative | Negative | Negative |
| 7 | Negative | Positive | Negative |
| 8 | Negative | Negative | Negative |
| 9 | Negative | Negative | Negative |
| 10 | Negative | Negative | Negative |
| 11 | Negative | Negative | Negative |
| 12 | Negative | Negative | Negative |
| 13 | Negative | Negative | Negative |
| 14 | Negative | Negative | Negative |
| 15 | Negative | Negative | Negative |
| 16 | Negative | Negative | Negative |
| 17 | Negative | Negative | Negative |
| 18 | Negative | Negative | Negative |
| 19 | Negative | Negative | Negative |
| 20 | Negative | Negative | Negative |

**Table IX: Mascot search results for the identified protein peptides.**

| **Name of peptide sequence** | **Accession number** | **Mass of peptide sequence/Da** | **Protein sequence coverage/%** | **Score** | **No. of peptides with expected value < 0.2** |
|---|---|---|---|---|---|
| **A2MG HUMAN** | P01023 | 163188 | 12% | 409 | 12 |
| **FIBA HUMAN** | P02671 | 94914 | 23% | 694 | 11 |
| **AACT HUMAN** | Q6NSC9 | 47621 | 8% | 176 | 2 |
| **CO3 HUMAN** | P01024 | 187030 | 4% | 174 | 4 |
| **FIBB HUMAN** | P02675 | 55892 | 39% | 853 | 11 |
| **FIBG HUMAN** | Q9UC63 | 51479 | 33% | 381 | 10 |
| **A1AT HUMAN** | Q86U18 | 46707 | 28% | 352 | 7 |
| **APOA1 HUMAN** | P02647 | 30759 | 31% | 334 | 7 |

### Effect of addition of specific antibodies to serum samples in SDS-page, SPR and mass spectrophotometry

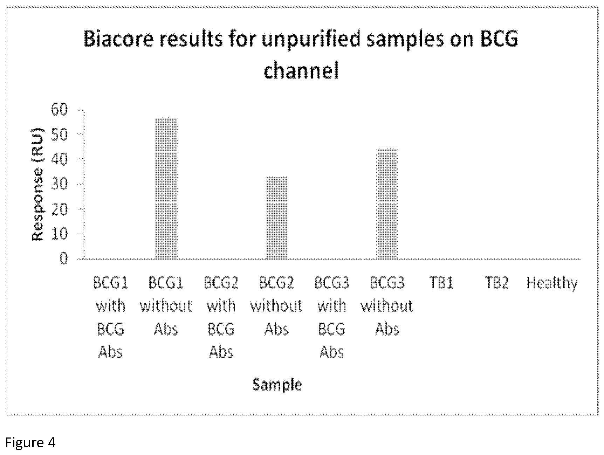

In order to understand which particles/peptides in blood interact with the produced antibodies we performed analysis on blood with and without antibodies.
SPR: Albumin and IgG depleted serum samples of TB patients (n=2), healthy control (n=1) and BCG vaccinated person (n=1) to which either Mtb antibodies or BCG antibodies have been added and incubated at RT for 1 hour or without antibodies were run on Mtb channel in SPR. No response was recorded for any of the samples except TB sample without antibodies and with BCG antibodies (Fig. 3). In contrast, when BCG serum samples (n=3) with and without BCG antibodies, healthy (n=1) and TB (n=2) serum samples were run on BCG channel in SPR, only BCG samples without antibodies showed positive responses in SPR (Figure 4)

### SDS-PAGE

SDS-PAGE was performed to identify antigenic particles in the sera of TB patients that are specifically binding to our immobilized IgG antibodies. Albumin/IgG depleted serum samples of TB patients to which Mtb antibodies were added and incubated at RT for 1 hour produce no band on the gel (lanes 1-5 of Fig. 5A, 4-6 of Fig. 5B) and 1-4 on Fig. 5C) whilst those without antibodies produced bands at 38kD, 65kD (lanes 6-9 of Fig. 5A and lanes 1-3 of Fig. 5B respectively) and between 30kD and 250kD (Fig. 5C). Serum samples from healthy individuals showed some bands (lanes 5-9 of Fig. 5C).

### Mass spectrophotometry

Four bands of TB samples obtained at 165kD, 65kD, 38kD and 30kD and that do not match bands of healthy controls were selected for LC-MS/MS analysis to characterize the proteins. Bands of healthy control (lanes 5-9 of Fig. 5C) were not selected since they did not bind to our Mtb antibodies in SPR. MGF files of protein fragments that were produced after reading samples in LC-MS/MS were used to search for proteins in Swiss-Prot from Mascot. Peptides were considered reliable if the MS/MS spectra had Mascot scores above 25 and expected value less than 0.2 (Table IX) and were selected. Names of the peptides and their bioinformatic data can be found in Table IX. Alpha 2 Macroglobulin is a human peptide that matched more accurately (95%) with the peptides that were found and interacted with our specific antibodies.
The nature of produced antibodies was determined with ELISA (Human IgG subclass analysis Invitrogen AB) and SPR. The antibodies had high affinity to anti human IgG in SPR and mostly consisted of IgG subclass.

**Table X: The antibodies produced were of IgG all subclasses.**

| ELISA for | Mtb abs | |
|---|---|---|
| Id | sample (conc µg/ml) | control(conc µg/ml) |
| IgG 1 | 479.78 | 136.51 |
| IgG 2 | 71.75 | 28.88 |
| IgG3 | 84.42 | 32.21 |
| IgG4 | 16.46 | 3.74 |

### Analysis of serum samples of patients and controls in SPR

Analysis of serum samples of patients (n=13), vaccinated personal (n=19) and healthy blood donors (n=27) in SPR in both MTB (Mycobacterium tuberculosis) flow cell and BCG flow cell could differentiated BCG vaccination from disease with high sensitivity and specificity > 95%.

**Table XI: The sensitivity and specificity of Monoman antibodies to differentiate BCG vaccinated individuals from healthy Swedish blood donors and Tuberculosis verified or suspect/treated tuberculosis patients > 95%.**

| | Healthy blood donors (n=27) | | BCG vaccinated personal (n=19) | | Tuberculosis verified non treated (n=5) | | Tuberculosis high susp (n=8) or after initiated therapy | |
|---|---|---|---|---|---|---|---|---|
| | BCG | MTB | BCG | MTB | BCG | MTB | BCG | MTB |
| Positive < 100 RU | 0 | 0 | 13 | 0 | 0 | 0 | 0 | 8 |
| Positive > 100 RU | 0 | 0 | 6 | 0 | 0 | 5 | 0 | 0 |
| Negative | 27 | 27 | 0 | 19 | 0 | 0 | 0 | 0 |
| Total | 27 | 27 | 19 | 19 | 0 | 5 | 0 | 8 |

### Vaccination option:

The most extensively studied protein among those identified in our study is the A2M. Previous studies about this tetrameric glycoprotein in the context of TB provide a great tendency for further research. This 740kD protease inhibitor that is present in virtually all vertebrates has been shown to be immunogenic. It is evolutionarily conserved arm of the immune system. They bind to their receptor, CD91 (LRP/A2MR), on APCs and enhance the uptake, processing and presentation of peptides they have chaperoned. Binding to CD91 also leads to the production of peptide-specific CD8+ T cell responses in mice immunized with A2M-peptide complex. A2M are also known to bind several cytokines and proteases. A2M-antigen complexes produce strong antibody response and a CD4+ T cell response after their internalization by APCs. Binder RJ (2003) has shown that purified A2M could be conjugated with peptides from infectious agent for use in vaccine production. The use of A2M-peptide complex for tumor prophylaxis has already been described by this same author. In an *in vitro* study conducted by Chu *et al.* (1994), there was 10 to 500-fold increase in IgG production when A2M was conjugated with antigen compared to unconjugated controls. This lead to their proposition that A2M may enhance antibody production and antigen uptake by macrophage.

### Discussion

Herein, the interaction and binding affinity of specific antibodies produced by Mtb and BCG stimulated B cells in SPR system are presented.

During the stimulation of B cells in the culture medium to produce antibodies against Mtb, morphological differences were observed in the cells under microscope. Cells were seen to substantially increase in sizes and numbers and later aggregated. B cells become activated and differentiate when induced to produce antigen-specific antibodies.²⁵ Smith *et al.*²⁶ had reported that antigen-specific B cells increase in numbers in the GC within the first two weeks of immunization but the number starts to decline after the second week until they are totally depleted after day 21. Thus antibodies were extracted exactly after two weeks of incubation.²⁶

Immobilization of antibodies onto the biacore sensor chip was performed immediately after the extraction process. The binding affinity of the antibodies produced from Mtb antigen was much greater than that of BCG vaccine. Specificity tests of the chip indicate that antibodies that were produced during the study were very specific as they bound specifically to positive controls used in each of the channels (Fig. 1A-C). This was as a result of the fact that the B cells were stimulated with Mtb from the same patient. The presence of IgG antibodies produced was confirmed by running SDS-PAGE. We obtained bands at 50kDa (heavy chain) and 25kDa (light chain). Memory B cells respond to specific antigen in secondary infection by producing antigen-specific IgG^{24, 26}, and here, as described above, the medium was stimulated with Mtb previously found in cultures of the patient's blood.

Biacore samples were detected in response units (RU) which were depicted when different concentrations of samples diluted in HBS were injected on the sensor surface immobilized with their respective antibodies. Furthermore, results in the SPR analysis were compared with culture results of the TB and pneumonia serum and plasma samples. None of the healthy and pneumonia samples responded positively to the antibodies produced from Mtb antigen, indicating that they do not have Mtb infection, as confirmed by the culture results (Table VII and VIII). Conversely, one of the healthy samples (Fig. 3B) and most of the pneumonia samples (Table VII and VIII, Fig. 3A) responded positively to the antibodies produced from BCG vaccine. This may be due to the fact that these individuals had been vaccinated against Mtb.

In the culture results (Fig. 3D) all TB samples were positive and all healthy samples were culture negative as confirmed by the biacore results (Table VIII). Pneumonia samples had 6 culture positive and 4 culture negative results respectively but none of them was Mtb positive. Not all the TB samples responded positively to antibodies on BCG channels (Fig. 2 and 3C) and this may be associated with specificity.

### Conclusion

Antibodies that are specific to Mtb and BCG have been produced in vitro. They can be used in a method for detecting whether an individual has a tuberculosis infection and/or whether an individual has been vaccinated against tuberculosis. The method may for example be employed for detecting latent tuberculosis infection (LTBI).

### References

1. World Health Organization Report (2009). Global tuberculosis control: epidemiology, strategy,financing. Accessed on 16th November, 2011 at: http://www.who.int/tb/publications/global_report/2009/pdf/report_without_annexes.pdf.
2. Stop TB Partnership. **The Global Plan to Stop TB 2011-2015.** Geneva. Accessed on 16th November, 2011 at: www.stoptb.org/.
3. Raja A. (2004). Immunology of tuberculosis. Indian J Med Res 120(4): 213-232.
4. Tundup S, Pathak N, Ramanadham M, Mukhopadhyay S & Murthy K. J. R. (2008). The Co-Operonic PE25/PPE41 Protein Complex of Mycobacterium tuberculosis Elicits Increased Humoral and Cell Mediated Immune Response. PLoS ONE 3(10): e3586.
5. Abu-Raddada L. J, Sabatelli L., Achterberga J. T., Sugimoto J. D., Longini I. M. Jr, Dyee C., and Halloran M. E.. (2009) Epidemiological benefits of more-effective tuberculosis vaccines, drugs, and diagnostics. PNAS 106 (33): 13980-13985.
6. Global tuberculosis control. WHO report 2011. Geneva. Accessed on 16th November, 2011 at: http://www.who.int/tb/publications/global report/2011/gtbr11 full.pdf
7. Hewinson R. G. (2005) TB vaccines for the World. Tuberculosis 85: 1-6.
8. Agger E. M. and Andersen P. (2002) A novel TB vaccine; towards a strategy based on our understanding of BCG failure. Vaccine 21: 7-14.
9. Rajni and Meena L. S. (2011) Unique Characteristic Features of Mycobacterium tuberculosis in Relation to Immune System. American Journal of Immunology 7 (1): 1-8.
10. Kaufmann S. H. E. and Hess J. (2000) Immune response against Mycobacterium tuberculosis: implications for vaccine development. Journal of Biotechnology 83: 13-17.
11. Pieters J (2008). Mycobacterium tuberculosis and the macrophage: maintaining a balance. Cell Host & Microbe 3(6):399-407.
12. Boom W. H., Canaday D. H., Fulton S. A., Gehring A. J., Rojas R. E., and Torres M.. (2003) Human immunity to M. tuberculosis: T cell subsets and antigen processing. Tuberculosis 83, 98-106.
13. Grange J. M., Brunet L. R., and Rieder H. L. (2011) Immune protection against tuberculosis - When is immunotherapy preferable to vaccination? Tuberculosis 91: 179e185.
14. Parida S. K. and Kaufmann S. H. E. (2010) Novel tuberculosis vaccines on the horizon. Current Opinion in Immunology 22:374-384.
15. Beresford B. and Sadoff J. C. (2010) Update on Research and Development Pipeline: Tuberculosis Vaccines. Clinical Infectious Diseases 50(S3):S178-S183.
16. Swaminathan S. and Rekha B. (2010) Pediatric tuberculosis: Global Overview and Challenges. Clinical Infectious Diseases 50(53): S184-S194.
17. de Vallière S., Abate G., Blazevic A., Heuertz R. M. and Hoft D. F. (2005) Enhancement of Innate and Cell-Mediated Immunity by Antimycobacterial Antibodies. Infect. Immun 73(10):6711.
18. Paul J. Maglione and Chan J. (2009) How B cells shape the immune response against Mycobacterium tuberculosis. Eur. J. Immunol. 39: 676-686.
19. Cooper A. M. (2009). Cell-Mediated Immune Responses in Tuberculosis. Annu. Rev. Immunol. 27:393-422.
20. Hoft D. F. (2008) Tuberculosis vaccine development: goals, immunological design, and evaluation. Lancet 372: 164-75.
21. Abebe F. and Bjune G. (2009) The protective role of antibody responses during Mycobacterium tuberculosis infection. Clinical and Experimental Immunology, 157: 235-243.
22. Dheda K., Schwander S. K., Zhu B., Van Zyl-Smit R. N. and Zhang Y. (2010) The immunology of tuberculosis: From bench to bedside. Respirology 15(3):433-450.
23. Glatman-Freedman A. (2006) The role of antibody-mediated immunity in defense against Mycobacterium tuberculosis: Advances toward a novel vaccine strategy. Tuberculosis 86:191-197.
24. McHeyzer-Williams L. J., Cool M. and McHeyzer-Williams M. G. (2000) Antigen-specific B Cell Memory: Expression and Replenishment of a Novel B220-Memory B Cell Conpartment. J. Exp. Med. 191(7): 1149-1165.
25. Oshiba A. and Gelfand E. W. (1999) Engagement of B-cell antigen receptor by antigen negatively regulates IgE production by antigen-specific B cells. J Allergy Clin Immunol 103:341-348.
26. Smith K. G. C., Light A. Nossal G. J. V. and Tarlinton D. M. (1997) The extent of affinity maturation differs between the memory and antibody-forming cell compartments in the primary immune response. The EMBO Journal 16(11): 2996-3006.
27. Kodituwakku A. P., Jessup C., Zola H. and Roberton D. M. (2003) Isolation of antigen-specific B cells. Immunology and Cell Biology 81:163-170.
28. Karlsson R. (2004) SPR for molecular interaction analysis: a review of emerging application areas. J. Mol. Recognit 17: 151-161.
29. Besenicar M., Macek P., Lakey J. H. and Anderluh G. (2006) Surface plasmon resonance in protein-membrane interactions. Chemistry and Physics of Lipids 141: 169-178.
30. Daghestani H. N. and Day B. W. (2010) Theory and Applications of Surface Plasmon Resonance, Resonant Mirror, Resonant Waveguide Grating, and Dual Polarization Interferometry Biosensors. Sensor 10:9630-9646.
31. Baoxia L., Juan C. & Mian L. (2008) Quantifying cell binding kinetics mediated by surface-bound blood type B antigen to immobilized antibodies. Chinese Science Bulletin 53(23): 3634-3641.
32. Rich R. L. and Myszka D. G. (2000) Advances in surface plasmon resonance biosensor analysis. Current Opinion in Biotechnology 11:54-61.
33. Hearty S., Conroy P. J., Ayyar B. V., Byrne B., and O'Kennedy R. (2010) Surface plasmon resonance for vaccine design and efficacy studies: recent applications and future trends. Expert Rev. Vaccines 9(6): 645-664.
34. Waswa J. W., Debroy C. and Irudayaraj J. (2006) Rapid Detection of Salmonella Enteritidis and Escherichia Coli using Surface Plasmon Resonance Biosensor. Journal of Food Process Engineering 29: 373-38.
35. Jongerius-Gortemaker B.G., Goverde R.L., van Knapen F. and Bergwerff A.A. (2002) Surface plasmon resonance (BIACORE) detection of serum antibodies against Salmonella enteritidis and Salmonella typhimurium. J Immunol Methods 266: 33-44.
36. O'Shannessey D.J., Brigham-Burke M. and Peck K. (1992) Immobilization chemistries suitable for use in the BIAcore surface Plasmon resonance detector. Anal. Biochem. 205: 132-136.

## Claims

1. An *in vitro* method for differentiating individuals having active *Mycobacterium tuberculosis* (Mtb) infection, individuals having a latent Mtb infection, and individuals being healthy or vaccinated against *tuberculosis* infection, comprising the steps
- contacting a serum or plasma sample from said individual with an antibody produced by contacting heat-killed *Mycobacterium tuberculosis* bacteria with lymphocytes isolated from a host previously exposed to *Mycobacterium tuberculosis* bacteria, wherein the antibody is an IgG antibody, has specificity for a Mtb protein and shows cross-reactivity with a protein selected from the proteins of the following list:
A2MG_HUMAN
FIBA_HUMAN
AACT_HUMAN
C03_HUMAN
FIBB_HUMAN
FIBG_HUMAN
A1AT_HUMAN, and
APOA1_HUMAN;
and
- studying the interaction between the proteins in the sample and the antibody in comparison to healthy controls,
wherein high levels of the proteins interacting with the antibody are indicative of an active Mtb infection, low levels of the proteins interacting with the antibody are indicative of a latent Mtb infection, and no levels of the proteins interacting with the antibody are indicative of a vaccinated or healthy individual.

2. An *in vitro* method for detecting whether an individual has a tuberculosis infection, comprising:
- contacting a serum or plasma sample from the individual with an antibody having a specificity for *Mycobacterium tuberculosis* (Mtb), which antibody has been produced by contacting heat-killed *Mycobacterium tuberculosis* bacteria with lymphocytes isolated from a host previously exposed to *Mycobacterium tuberculosis* bacteria, wherein the antibody is an IgG antibody, has specificity for a Mtb protein and shows cross-reactivity with a protein selected from the proteins of the following list:
A2MG_HUMAN
FIBA_HUMAN
AACT_HUMAN
CO3_HUMAN
FIBB_HUMAN
FIBG_HUMAN
A1AT_HUMAN, and
APOA1_HUMAN;
and
- studying the interaction between the proteins in the sample and the Mtb antibody in comparison to healthy controls,
wherein the presence of an interaction between the proteins in the sample and the Mtb antibody indicates that the individual has a tuberculosis infection.

3. The *in vitro* method of claim 2, further comprising detecting whether the individual has been vaccinated against tuberculosis infection, by:
- contacting the sample from the individual with an antibody having a specificity for the Bacille-Calmette-Guerin (BCG) vaccine, which antibody has been produced by contacting a heat-killed BCG vaccine with lymphocytes isolated from a host previously vaccinated with a BCG vaccine, wherein the antibody is an IgG antibody and
- studying the interaction between the sample and the BCG antibody in comparison to healthy controls,
wherein the presence of an interaction between the sample and the BCG antibody indicates that the individual has been vaccinated against tuberculosis infection.

4. The *in vitro* method of any of claims 2-3 wherein the infection is a latent tuberculosis infection.

5. The *in vitro* method of any of claims 1-4 wherein the antibody is immobilised to a solid surface and the interaction between the sample and the antibody is studied by use of surface plasmon resonance (SPR).

## Patentansprüche

1. *In-vitro*-Verfahren zum Differenzieren von Individuen, die eine aktive *Mycobacterium-Tuberkulosis* (Mtb) -Infektion aufweisen, Individuen, die eine latente Mtb-Infektion aufweisen und Individuen, die gesund sind, oder gegen eine Tuberkulose-Infektion geimpft sind, umfassend die Schritte
- Kontaktieren einer Serum- oder Plasmaprobe des Individuums mit einem Antikörper, hergestellt durch Kontaktieren von hitzegetöteten *Mycobacterium-Tuberculosis-*Bakterien mit Lymphozyten, isoliert aus einem zuvor *Mycobacterium-Tuberculosis*-Bakterien ausgesetzten Wirt, wobei der Antikörper ein IgG-Antikörper ist, der eine Spezifität für ein Mtb-Protein aufweist und eine Kreuzreaktivität mit einem Protein zeigt, ausgewählt aus den Proteinen der folgenden Liste:
A2MG_HUMAN
FIBA_HUMAN
AACT_HUMAN
CO3_HUMAN
FIBB_HUMAN
FIBG_HUMAN
A1AT_HUMAN und
APOA1_HUMAN;
und
- Untersuchen der Wechselwirkung zwischen den Proteinen in der Probe und dem Antikörper im Vergleich zu gesunden Kontrollen,
wobei hohe Pegel der mit dem Antikörper wechselwirkenden Proteine eine aktive Mtb-Infektion anzeigen, niedrige Pegel der mit dem Antikörper wechselwirkenden Proteine eine latente Mtb-Infektion anzeigen, und keine Pegel der mit dem Antikörper wechselwirkenden Proteine ein geimpftes oder gesundes Individuum anzeigen.

2. *In-Vitro*-Verfahren zum Nachweisen, ob ein Individuum eine Tuberkulose-Infektion aufweist, umfassend:
- Kontaktieren einer Serum- oder Plasmaprobe aus dem Individuum mit einem Antikörper, der eine Spezifität für *Mycobacterium Tuberculosis* (Mtb) aufweist, dessen Antikörper durch Kontaktieren von hitzegetöteten *Mycobacterium-Tuberculosis-*Bakterien mit Lymphozyten hergestellt wurde, isoliert aus einem zuvor *Mycobacterium-Tuberculosis-*Bakterien ausgesetztem Wirt, wobei der Antikörper ein IgG-Antikörper ist, der eine Spezifität für ein Mtb-Protein aufweist und eine Kreuzreaktivität mit einem Protein zeigt, ausgewählt aus den Proteinen der folgenden Liste:
A2MG_HUMAN
FIBA_HUMAN
AACT_HUMAN
CO3_HUMAN
FIBB_HUMAN
FIBG_HUMAN
A1AT_HUMAN und
APOA1-HUMAN;
und
- Untersuchen der Wechselwirkung zwischen den Proteinen in der Probe und dem Mtb-Antikörper im Vergleich zu gesunden Kontrollen,
wobei das Vorhandensein einer Wechselwirkung zwischen den Proteinen in der Probe und dem Mtb-Antikörper anzeigt, dass das Individuum eine Tuberkulose-Infektion aufweist.

3. *In-vitro*-Verfahren nach Anspruch 2, ferner umfassend das Nachweisen, ob das Individuum gegen eine Tuberkulose-Infektion geimpft worden ist, durch:
- Kontaktieren der Probe des Individuums mit einem Antikörper, der eine Spezifität für den Bacille-Calmette-Guerin (BCG) -Impfstoff aufweist, dessen Antikörper durch Kontaktieren eines hitzegetöteten BCG-Impfstoffs mit Lymphozyten hergestellt wurde, isoliert aus einem zuvor mit einem BCG-Impfstoff geimpften Wirt, wobei der Antikörper ein IgG-Antikörper ist und
- Untersuchen der Wechselwirkung zwischen der Probe und dem BCG-Antikörper im Vergleich zu gesunden Kontrollen,
wobei das Vorhandensein einer Wechselwirkung zwischen der Probe und dem BCG-Antikörper anzeigt, dass das Individuum gegen eine Tuberkulose-Infektion geimpft worden ist.

4. *In-vitro*-Verfahren nach einem der Ansprüche 2-3, wobei die Infektion eine latente Tuberkulose-Infektion ist.

5. *In-vitro*-Verfahren nach einem der Ansprüche 1 bis 4, wobei der Antikörper an einer festen Oberfläche immobilisiert ist und die Wechselwirkung zwischen der Probe und dem Antikörper unter Verwendung von Oberflächenplasmonenresonanz (SPR) untersucht wird.

## Revendications

1. Procédé *in vitro* de différenciation d'individus atteints d'une infection active par *Mycobacterium tuberculosis* (Mtb), d'individus atteints d'une infection latente par Mtb, et d'individus en bonne santé ou vaccinés contre une infection par la tuberculose, comprenant les étapes consistant à
- mettre en contact un échantillon de sérum ou de plasma provenant dudit individu avec un anticorps produit en mettant en contact des bactéries de *Mycobacterium tuberculosis* tuées par la chaleur avec des lymphocytes isolés d'un hôte exposé au préalable à des bactéries de *Mycobacterium tuberculosis,* dans lequel l'anticorps est un anticorps IgG, présente une spécificité pour une protéine de Mtb et présente une réactivité croisée avec une protéine choisie parmi les protéines de la liste suivante :
A2MG_HUMAN
FIBA_HUMAN
AACT_HUMAN
CO3_HUMAN
FIBB_HUMAN
FIBG_HUMAN
A1AT_HUMAN et
APOA1_HUMAN
et
- étudier l'interaction entre les protéines dans l'échantillon et l'anticorps par comparaison avec des témoins en bonne santé,
dans lequel des niveaux élevés des protéines interagissant avec l'anticorps indiquent une infection active par Mtb, des niveaux faibles des protéines interagissant avec l'anticorps indiquent une infection latente par Mtb, et des niveaux nuls des protéines interagissant avec l'anticorps indiquent un individu vacciné ou en bonne santé.

2. Procédé *in vitro* permettant de détecter si un individu est atteint par une infection par la tuberculose, consistant à :
- mettre en contact un échantillon de sérum ou de plasma provenant de l'individu avec un anticorps présentant une spécificité pour *Mycobacterium tuberculosis* (Mtb), ledit anticorps ayant été produit en mettant en contact des bactéries de *Mycobacterium tuberculosis* tuées par la chaleur avec des lymphocytes isolés d'un hôte exposé au préalable à des bactéries de *Mycobacterium tuberculosis,* dans lequel l'anticorps est un anticorps IgG, présente une spécificité pour une protéine de Mtb et présente une réactivité croisée avec une protéine choisie parmi les protéines de la liste suivante :
A2MG_HUMAN
FIBA_HUMAN
AACT_HUMAN
CO3_HUMAN
FIBB_HUMAN
FIBG_HUMAN
A1AT_HUMAN et
APOA1_HUMAN
et
- étudier l'interaction entre les protéines dans l'échantillon et l'anticorps de Mtb par comparaison avec des témoins en bonne santé,
dans lequel la présence d'une interaction entre les protéines dans l'échantillon et l'anticorps de Mtb indique que l'individu est atteint d'une infection par la tuberculose.

3. Procédé *in vitro* selon la revendication 2, consistant en outre à détecter si l'individu a été vacciné contre une infection par la tuberculose, en :
- mettant en contact l'échantillon provenant de l'individu avec un anticorps ayant une spécificité pour le vaccin de Bacille-Calmette-Guérin (BCG), ledit anticorps ayant été produit en mettant en contact un vaccin de BCG tué par la chaleur avec des lymphocytes isolés d'un hôte vacciné au préalable avec un vaccin de BCG, dans lequel l'anticorps est un anticorps IgG et
- étudiant l'interaction entre l'échantillon et l'anticorps de BCG par comparaison avec des témoins en bonne santé,
dans lequel la présence d'une interaction entre l'échantillon et l'anticorps de BCG indique que l'individu a été vacciné contre une infection par la tuberculose.

4. Procédé *in vitro* selon l'une quelconque des revendications 2 à 3, dans lequel l'infection est une infection latente par la tuberculose.

5. Procédé *in vitro* selon l'une quelconque des revendications 1 à 4, dans lequel l'anticorps est immobilisé sur une surface solide et l'interaction entre l'échantillon et l'anticorps est étudiée en utilisant une résonance plasmonique de surface (SPR).
